# EUROPEAN PATENT APPLICATION

(11) **EP 1 602 928 A1**
(43) Date of publication of application: **07.12.2005**
(21) Application number: 04076608.1
(22) Date of filing: 01.06.2004
(51) Int. Cl.: G01N 33/543, G01N 33/539

(54) **Process and kit for determining binding parameters of bioaffinity binding reactions**

(71) Applicant: Universiteit Maastricht, 6200 MD Maastricht (NL)
(72) Inventor: Hermens, Willem Theodoor, 6247 CV Gronsveld (NL); Speijer, Johan Gerhard, 6367 BJ Voerendaal (NL)
(74) Representative: Valkonet, Rutger

(57) **Abstract**

A process for determining binding parameters, including dissociation constants and sorption rate constants, of the binding between a biomolecule and a binding partner thereof, both being present in a liquid phase, and comprising a marker-free binding step, characterised by the following steps: (a) attaching either (a1) the binding partner, or (a2) the biomolecule, to a solid surface (b) allowing binding between the binding partner and the biomolecule, both in the liquid phase and on the solid surface (c) attaching a marker to: (c1) the biomolecule, if said binding partner was attached to the solid surface according to step (a1), or to (c2) the binding partner, if said biomolecule was attached to the solid surface according to step (a2) (d) allowing the marker to produce a precipitate; and (e) detecting said precipitate as it is formed on said solid surface by determining a change of surface mass on the solid surface due to the formation of said precipitate.

## Description

The present invention relates to a process for determining binding parameters, including dissociation constants and sorption rate constants, of the binding between a biomolecule and a binding partner thereof, both being present in a liquid phase, and comprising a marker-free binding step. The invention also relates to a slide for carrying out the process, it also relates to a kit for carrying out the process, which comprises at least a solid surface, consisting of a slide according to the invention.

Processes for determining binding parameters of the marker-free binding between biomolecules and their binding partners are generally known in the art. A relevant review is provided by Haake, H.M., Schütz, A. en Gauglitz, G., Fresenius J. Anal. Chem. (2000) 366:576-585.

As it is generally known in the state of the art, the dissociation constant (abbreviated as K_{d}) of a particular binding is defined as the ratio between the so-called "on-sorption rate constant" (abbreviated as kₒₙ) and the "off-sorption rate constant" or desorption rate constant (abbreviated as k_{off}), which can be expressed by the equation K_{d} = k_{off}/kₒₙ.

In the context of the present invention, the term "biomolecule" is defined as a molecule with a biological origin, such as, but not limited to, a protein, nucleic acid, lipid, carbohydrate, steroid and prostaglandin, which is formed in vivo or in vitro, and, in the latter case, is either identical or equivalent to the natural form of the biomolecule, which biomolecule usually, but not necessarily, has a biological or physiological activity.

In the context of the present invention, the term "binding partner" is defined as a molecule having an affinity, and to a certain degree also specificity, for a particular biomolecule. The binding partner usually, but not necessarily, is a low molecular weight organic compound (e.g. a steroid, where the biomolecule is a cell surface receptor protein; or an organic substrate molecule, where the biomolecule is an enzyme); however, it can itself also be a biomolecule (e.g. an antibody, which is essentially a protein, where the biomolecule is an antigen, which antigen can itself be a protein).

Essentially, the prior art methods measure the binding of a biomolecule (such as an antibody) on solid surfaces coated with a binding partner for a biomolecule (such as an antigen) in buffer solutions. Usually, the surface is covered with e.g. the biomolecule, either by covalent coupling or by physical adsorption. The value of K_{d} is then estimated for a series of increasing concentrations of the binding partner, essentially as the concentration for which binding is half-maximal. Alternatively, the value for the rate constant of adsorption (kₒₙ) is estimated from the initial adsorption phase, and the value for the rate constant of desorption (k_{off}) is then estimated by removing the binding partner (e.g. by washing or flushing) and in some way measuring the desorption.

However, several disadvantages are associated with the prior art processes for determining binding parameters, and particularly so if the K_{d} values involved are lower than 10⁻⁹ M. Firstly, even for transport-limited adsorption of the binding partner (which is usually not attained), the adsorption of the binding partner will in practice take too much time when binding partner concentrations become as low as 10⁻¹⁰ M - 10⁻¹¹ M. Secondly, due to the high kₒₙ values involved, the renewed adsorption of binding partner during flushing or washing (which is required for each concentration of biomolecule or binding partner) is significant, and the so-called "apparent k_{off}" (abbreviated as k_{off,app}) becomes much lower than the value of the true k_{off}. Furthermore, weak and aspecific interactions of adsorbed binding partner molecules with the surface may undesirably interfere with desorption, even to the point that the binding becomes virtually irreversible.

There is a need for a process which is less, or preferably not, subject to the aforementioned limitations concerning measurements when the dissociation constants are lower than 10⁻⁹ M. Furthermore, there is a need for a process for determining binding parameters of the binding between a biomolecule and a binding partner thereof which comprises fewer steps, and is therefore less laborious and cumbersome than the prior art method.

The process for determining binding parameters according to the present invention is characterised by the following steps:
(a) fixating either (a1) the binding partner, or (a2) the biomolecule, to a solid surface;
(b) allowing binding between the binding partner and the biomolecule, both in the liquid phase and on the solid surface;
(c) attaching a marker to: (c1) the biomolecule, if said binding partner was fixated to the solid surface according to step (a1), or to (c2) the binding partner, if said biomolecule was fixated to the solid surface according to step (a2);
(d) allowing the marker to produce a precipitate; and
(e) detecting said precipitate as it is formed on said solid surface by determining a change of surface mass on the solid surface due to the formation of said precipitate.

The solid surface is used as a measuring tool when measuring the binding equilibrium in the liquid phase. The binding partner or the biomolecule is fixated to a pretreated solid surface (step a). The amount of binding to the surface by the binding partner (or, respectively, the biomolecule) is a measure for the free concentration of the binding partner (or, respectively, the biomolecule) in the liquid phase (step b). Amplification of the signal is necessary for measuring the often very low concentrations, which is achieved by attaching a marker to the biomolecule (or, respectively, to the binding partner) (step c). The binding as such is marker-free (thus avoiding marker-induced changes of binding affinities and a possible error in the estimation of the dissociation constant).The marker is then allowed to produce a precipitate (step d) and the velocity of the formation of said precipitate on the solid surface is detected by determining a change of surface mass on the solid surface due to the formation of said precipitate.

An advantage of the present process is that, as the precipitate is detected by determining a change of surface mass on the solid surface, only the substrate converted directly at the surface will contribute to precipitate formation. Small aggregates of converted substrate in the liquid phase will not interfere with the measurement. This actually leads to a so-called one-step assay process for determining binding parameters, without the cumbersome and laborious intervening washing or flushing steps which are obligatory when using prior art processes. According to the present invention, equilibrium is obtained fairly quickly. The free binding partner (or free biomolecule) concentration can be determined by comparing the rate of the formation of the precipitate with a standard curve. From the free concentrations thus measured, the binding constants are then calculated. A major advantage of the process according to the invention is that the free concentrations can be measured while in equilibrium with complexes of the biomolecule and the binding partner thereof. The above-mentioned aspecific interactions, as well as the problems involved with differences between true sorption constants and apparent sorption constants, which may cause considerable errors, are therefore avoided when using the process according to the invention. Finally, and as will be illustrated hereinafter, the surface area of the present solid surface according to the invention can be kept very small, thereby preventing any significant influence of protein adsorption on the free protein concentration.

A preferred embodiment of the present invention relates to a process for determining binding parameters, in which the biomolecule is an enzyme and the binding partner is an inhibitor or a substrate therefor. According to another preferred embodiment, the biomolecule is a cell membrane protein and the binding partner is ligand therefor. Preferably, said cell membrane protein is a receptor. According to another embodiment of the present invention, the biomolecule is a cell membrane protein and the binding partner is a cell membrane structure. Preferably, said cell membrane structure is artificial, and according to a particular embodiment, the cell membrane structure is a complete cell.

A preferred variant of the present invention relates to a process in which the biomolecule is an antibody or a fragment thereof, and the binding partner is an antigen of said antibody or fragment. Preferably, the marker of the present process is an enzyme producing a precipitate from a soluble substrate.

Particularly preferred embodiments of the present solid surface are (i) a silicon slide, and (ii) a chromium-sputtered glass.

Although step (e) of the present process can be carried out in several ways, it is preferred that the determination of the change of surface mass is carried out by ellipsometry. However for carrying out the method according to the invention other analysing techniques for determining of the change of surface mass can be implemented, for example an analysing technique based on the principle of Surface Plasmon Resonance (SPR) or Total Internal Reflection Fluorescence (TIRF). With both techniques (SPR and TIRF), but in particular with the SPR-technique similar results can be achieved.

Preferably, the step of determining a change of surface mass is carried out on the surface area of less than 3.0 mm².

According to a particular embodiment of the present process, the dissociation constant is lower than 10⁻⁹ M, and is in particular 10⁻⁹ M - 10⁻¹³ M.

The present invention also relates to a slide for carrying out the present process. The slide should be covered with a protein-adsorbing layer, preferably a synthetic polymer. Preferably, the slide is cut from a phosphorous-doped and silica-coated silicon wafer, and is covered with a PVC layer or a polystyrene layer. It is preferred that the PVC layer has a thickness of 10-30 nm. According to another preferred embodiment of the aforementioned slide, it is covered with a silane layer, which preferably has a thickness of 1-5 nm. Slides can be modified in order to enable attachment of a biomolecule or binding partner. A preferred slide is characterised in that it is cut from a phosphorous-doped and silica-coated silicon wafer and is modified by attachment of a molecule with an amino group. The amino group is then available for covalent binding of the biomolecule or binding partner thereof, by a treatment with N-succinimidyl-3-(2-pyridyldithio)proprionate (SPDP).

Finally, the present invention also relates to a kit for carrying out the process according to the present invention, comprising at least a solid surface consisting of silicon slides or chromium sputtered glass, and either a marked binding partner or a marked biomolecule. Preferably, said kit also contains a standard of the binding partner or the biomolecule to be determined. The present invention also relates to a kit for carrying out the process according to the invention, comprising at least a solid surface capable of immobilising the biomolecule or its binding partner, or with the biomolecule or its binding partner already bound. In a particular embodiment the biomolecule or its binding partner is a murine monoclonal antibody, and the marker is a HRP-labelled secondary antibody which binds to mouse monoclonal antibodies. The kit preferably comprises at least a solid surface consisting of silicon slides or chromium sputtered glass as well as a marker which binds to any antibody used as the binding partner of a biomolecule that was previously adsorbed on the slide. In particular embodiments, HRP-labelled Protein A or Protein G are used as a marker.

The present invention can be used to study different kinds of interactions, examples of which are (i) the interaction between a protein and a membrane (or membrane component), (ii) the interaction between an enzyme and an inhibitor thereof, (iii) the interaction between a membrane-bound receptor and a ligand for said receptor, and (iv) the interaction between an antibody and an antigen.

The invention will be illustrated by the following nonlimiting examples.
Figure 1 shows an example of the determination of the dissociation constant of an protein-membrane interaction;
Figure 2 shows an example of the determination of the dissociation constant of an enzyme-inhibitor interaction in the usual range of values;
Figure 3 shows the determination of the dissociation constant for the IL6-α126.16 complex in blood plasma, in the high affinity range of constants;
Figure 4a-4b show examples of the determination of binding constants of α-IL6.16.

### Example 1:

### Determination of the binding constants of a protein-membrane interaction

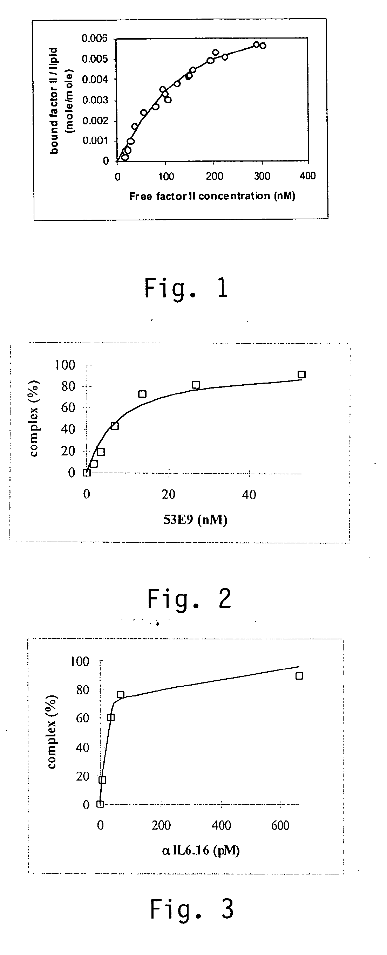

Small unilamellar vesicles (SUVs) were prepared by sonication of a phospholipid mixture of 20% dioleoyl-phosphatidylserine (DOPS) and 80% dioleoyl-phosphatidylcholine (DOPC). Various concentrations (25-500 nM) of purified bovine factor II were added to 0.05 mM (total phospholipid) SUVs in 0.05 M Tris-HCl buffer (pH 7.5), containing 0.1 M NaCl, 3 mM CaCl₂, 0.5 g/l bovine serum albumin, and 100 ng/ml of a rabbit anti-bovine factor II-HRP conjugate. Silica surfaces were covered with phospholipid bilayers by previous exposure of the slides to 0.020 mM SUVs. The free concentrations of factor II were measured by ellipsometry from the precipitation rates after addition of DAB-H₂O₂ substrate, using a commercially available ellipsometer (EL-X-02C, Dr. Riss Ellipsometerbau, GmbH, Ratzeburg, Germany). Results are shown in Figure 1 and a value of K_{d} = 123 ± 21 nM was obtained.

### Example 2:

### Determination of the dissociation constant for the FABP-53E9 complex in buffer

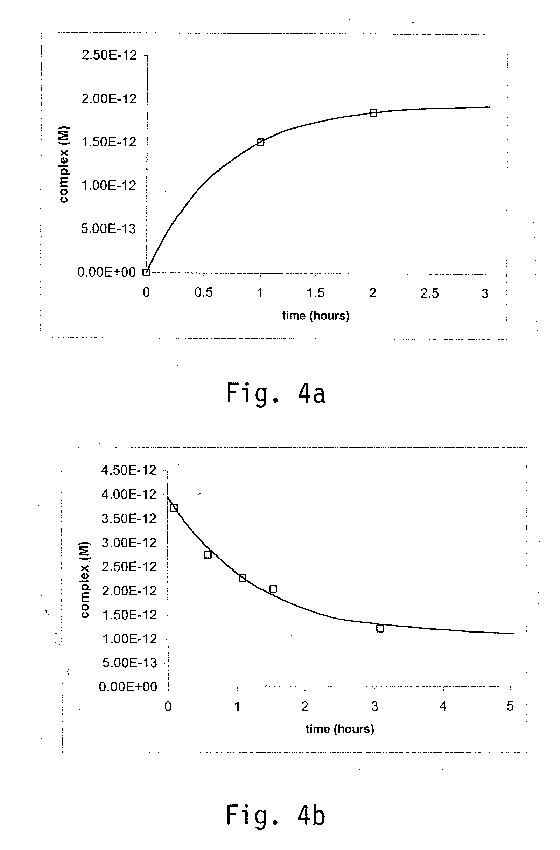

FABP was used as antigen and a monoclonal anti-FABP (53E9) was used as the corresponding antibody. Both were a kind gift of Prof. J. Glatz, Department of Physiology, CARIM. The dissociation constant K_{d} of this antibody has been reported to be 13,5 nM (Roos et al, J. Immunol. Meth. (1995) 183: 149-153). FABP (0,67 nM) was incubated during 30 minutes with various concentrations of antibody 53E9, until equilibrium in bulk solution was established (Fig. 2). Subsequently, the concentrations of free FABP were estimated by short (10 min) measurements of the rate of precipitation on silica surfaces with small surface areas covered with a 53E9 catcher, and another, HRP-marked, antibody against FABP (118 ng/ml). It is important that the equilibrium in the liquid phase is not disturbed by the latter procedure. Hence, without washing (one-step process), free FABP concentrations were measured from the rate of precipitate formation after addition of DAB-H₂O₂ substrate. The dissociation constant K_{d} of the FABP-53E9 interaction determined in this way was 8 nM.

### Example 3:

### Determination of the dissociation constant for the IL6-αIL6.16 complex in blood plasma

High-affinity anti-human IL6 antibodies were a kind gift of Prof. L. Aarden, Sanquin Research, Amsterdam. Plasma (5x diluted) was incubated during 60 minutes with various concentrations of the high-affinity monoclonal anti-human IL6 antibody aIL6.16, as indicated in Figure 3. The medium was contacted for an additional 120 min with an αIL6.16-coated silicon slide. Subsequently, a one-step process according to the invention was performed to determine the free IL6 concentration. The value of the dissociation constant K_{d} deduced from the simulated curve (Figure 3) was 24 pM.

### Example 4:

### Determination of binding constants of α-IL6.16

First IL6 (4 pM) was incubated with αIL6.16 (30 pM) for various time intervals, as indicated in Figure 4a. The medium was then incubated for an additional 10 minutes with an αIL6.16-coated silicon slide. Subsequently, the one-step process according to the invention was performed to determine the free IL6 concentration. K_{d}, kₒₙ and k_{off} as calculated from the simulated curve were 29 pM, 6.8 10⁶ M⁻¹s⁻¹ and 0,00020 s⁻¹, respectively.

Using a "reverse"process, IL6 (400 pM) was first preincubated with αIL6.16 (1 nM) for 50 minutes. The pre-incubation medium was then diluted 100-fold. After various time intervals, the medium was contacted for an addition 10 minutes with an αIL6.16-coated silicon slide. Subsequently, the one-step process according to the invention was carried out to determine the free IL6-concentration. K_{d}, kₒₙ and k_{off} from the simulated curve (Figure 4b) were 25 pM, 6.3 10⁶ M⁻¹s⁻¹ and 0,00016 s⁻¹, respectively.

### Example 5:

### Slides.

Several slides (5.1-5.4) were produced as follows.
5.1. Slides cut from phosphorus-doped and silica-covered silicon wafers were covered with PVC-layers with a thickness of 10-30 nm. This was done by retraction of the slide from a solution of PVC in cyclohexanone at controlled speeds, using a mechanical dipping device.
5.2. Slides cut from phosphorus-doped and silica-covered silicon wafers were covered with thin (1-5 nm) silane layers, using Sigmacote® and a protocol as indicated by the manufacturer.
5.3. Slides cut from phosphorus-doped and silica-covered silicon wafers were amino-terminated by immersion in a solution of (3-aminopropyl)trietoxysilane (Sigma) in acetone. Using the N-succinimidyl 3-(2-pyridyldithio)proprionate (SPDP) reagent, such slides were used for covalent binding of antibodies.
5.4. Streptavidin was covalently coupled to amino-terminated slides produced as described in 5.3. These slides were used for covalent binding of biotinylated antibodies.

### Example 6:

### Kits.

Kits (6.1. and 6.2.) were assembled as follows.
6.1. A kit comprising a suitable HRP-marked antibody was assembled as follows.
   Eight highly hydrophobic slides, prepared as described in Example 5 (5.1), together with HRP-marked 66E2 anti-human FABP (marker) a recombinant human FABP standard, blocking buffer, and precipitate forming HRP substrate (DAB+H₂O₂).
   To use this kit, the anti-FABP antibodies to be characterised should be coupled to the slides by overnight incubation in 2 mg/L of antibody at 4°C. The slides should then be incubated in blocking buffer. A known amount of FABP was preincubated for 30 minutes with five different concentrations of antibody and then added to ellipsometer cells, containing the slides. Using the one-step procedure according to the invention, including three calibrators, the amounts of free FABP can be measured. The kit will also contain two extra slides, to which FABP must be coupled, in order to check that the antibody to be characterised does not compete with the 66E2 anti-FABP tag.
6.2. A kit comprising a polyclonal anti-mouse IgG was assembled as follows.
   Eight slides of aforementioned types 5.1-5.4 (Example 5), allowing either passive or covalent coupling of the antigen to the slide, HRP-marked anti-mouse IgG (marker), blocking buffer, and precipitating HRP substrate (DAB + H₂O₂).
   To use this kit, the antigen used by the customer should be coupled to the slides and then incubated in blocking buffer. A fixed concentration of the antibody to be characterized should be pre-incubated with five different concentrations of antigen and then added to ellipsometer cells. Using the one-step procedure according to the invention, including three calibrators the amounts of free antibody can then be measured.

## Claims

1. A process for determining binding parameters, including dissociation constants and sorption rate constants, of the binding between a biomolecule and a binding partner thereof, both being present in a liquid phase, and comprising a marker-free binding step, **characterised by** the following steps:
(a) attaching either (a1) the binding partner, or (a2) the biomolecule, to a solid surface;
(b) allowing binding between the binding partner and the biomolecule, both in the liquid phase and on the solid surface;
(c) attaching a marker to: (c1) the biomolecule, if said binding partner was attached to the solid surface according to step (a1), or to (c2) the binding partner, if said biomolecule was attached to the solid surface according to step (a2);
(d) allowing the marker to produce a precipitate; and
(e) detecting said precipitate as it is formed on said solid surface by determining a change of surface mass on the solid surface due to the formation of said precipitate.

2. The process according to claim 1, **characterised in that** the biomolecule is an enzyme and the binding partner is an inhibitor or a substrate therefor.

3. The process according to claim 1, **characterised in that** the biomolecule is a cell membrane protein and the binding partner is a ligand therefor.

4. The process according to claim 3, **characterised in that** the cell membrane protein is a receptor.

5. The process according to claim 1, **characterised in that** the biomolecule is a cell membrane protein and the binding partner is a cell membrane structure.

6. The process according to claim 5, **characterised in that** the cell membrane structure is artificial.

7. The process according to claim 5 or claim 6, **characterised in that** the cell membrane structure is a complete cell.

8. The process according to claim 1, **characterised in that** the biomolecule is an antibody or a fragment thereof, and the binding partner is an antigen of said antibody or fragment.

9. The process according to any one of claims 1 to 8, **characterised in that** the marker is an enzyme producing a precipitate from a soluble substrate.

10. The process according to any one of claims 1 to 9, **characterised in that** the solid surface is a silicon slide.

11. The process according to any one of claims 1 to 9, **characterised in that** the solid surface is a chromium-sputtered glass.

12. The process according to any one of claims 1 to 11, **characterised in that** the determination of the change of surface mass of step (e) is carried out by ellipsometry.

13. The process according to any one of claims 1 to 12, **characterised in that** the step of determining a change of surface mass is carried out on a surface area of less than 3.0 mm².

14. The process according to any one of claims 1 tot 13, **characterised in that** the dissociation constant is lower than 10⁻⁹ M.

15. The process according to claim 14, **characterised in that** the dissociation constant is 10⁻⁹ M - 10⁻¹³ M.

16. A slide for carrying out a process according to any one of claims 1-10 or claims 12-15.

17. The slide according to claim 16, **characterised in that** the slide is cut from a phosphorus-doped and silica-coated silicon wafer, and is covered with a protein-adsorbing material.

18. The slide according to claim 17, in which the protein-adsorbing material is a synthetic polymer.

19. The slide according to claim 18, in which the synthetic polymer is PVC.

20. The slide according to claim 18, in which the synthetic polymer is polystyrene.

21. The slide according to claim 19, **characterised in that** the PVC layer has a thickness of 10-30 nm.

22. The slide according to claim 16, **characterised in that** the slide is cut from a phosphorus-doped and silica-coated silicon wafer, and is covered with a silane layer.

23. The slide according to claim 22, **characterised in that** the silane layer has a thickness of 1-5 nm.

24. The slide according to claim 16, **characterised in that** the slide is cut from a phosphorus-doped and silica-coated silicon wafer, and treated to introduce a chemically reactive group, enabling the chemical coupling of a biomolecule or a binding partner thereof.

25. The slide according to claim 24 in which the reactive group is an amino group.

26. The slide according to claim 25 in which the coupling is achieved by treatment with N-succinimidyl-3-(2-pyridyldithio)propionaat (SPDP).

27. A kit for carrying out a process according to any one of claims 1-15, comprising at least a solid surface consisting of silicon slides or chromium sputtered glass, and either a marked binding partner or a marked biomolecule.

28. The kit according to claim 27, **characterised in that** it also contains a standard of the binding partner or the biomolecule to be determined.

29. A kit for carrying out a process according to any one of the claims 1-15, comprising at least a solid surface consisting of silicon slides or chromium sputtered glass, as well as a marker which binds to any antibody used as the binding partner of a biomolecule that was previously adsorbed on the slide.

30. The kit according to claim 29, in which the marker is a HRP-labelled antibody reactive with the antibody used as the binding partner.

31. The kit according to claim 29, in which the marker is HRP-labelled Protein A or HRP-labelled Protein G.
